(19) **Europäisches Patentamt**
**European Patent Office**
**Office européen des brevets**

(11) Veröffentlichungsnummer: **0 620 725 B1**

(12) **EUROPÄISCHE PATENTSCHRIFT**

(45) Veröffentlichungstag der Patentschrift: **06.12.95**

(51) Int. Cl.6: **A61K 7/46**

(21) Anmeldenummer: **93901737.2**

(22) Anmeldetag: **07.01.93**

(86) Internationale Anmeldenummer:
**PCT/EP93/00014**

(87) Internationale Veröffentlichungsnummer:
**WO 93/13749 (22.07.93 93/18)**

(54) **VERFAHREN ZUR HERSTELLUNG VON STABILEN FLÜSSIGEN PARFÜMROHSTOFFMISCHUNGEN MIT HOHEM FESTSTOFFGEHALT.**

(30) Priorität: **10.01.92 DE 4200433**

(43) Veröffentlichungstag der Anmeldung:
**26.10.94 Patentblatt 94/43**

(45) Bekanntmachung des Hinweises auf die
Patenterteilung:
**06.12.95 Patentblatt 95/49**

(84) Benannte Vertragsstaaten:
**AT BE CH DE DK ES FR GB GR IE IT LI LU MC
NL PT SE**

(56) Entgegenhaltungen:
**DE-A- 3 922 389
DE-C- 144 002
US-A- 2 875 131
US-A- 3 548 006
US-A- 4 650 603**

**S.ARCTANDER 'Perfume and flavor chemicals' 1969 , S.ARCTANDER , MONTCLAIR,N.J.**

(73) Patentinhaber: **PROCTER AND GAMBLE CO.
1 Procter and Gamble Plaza
Cincinnati, OH (US)**

(72) Erfinder: **HOFSTETTER, Otto
Im Heimesgrund 1
D-6943 Birkenau (DE)**
Erfinder: **OSWALD, Gerhard
Carl-Goerdeler-Str. 38
D-6508 Alzey (DE)**

(74) Vertreter: **Grussdorf, Jürgen, Dr. et al
Patentanwälte Zellentin & Partner
Rubensstrasse 30
D-67061 Ludwigshafen (DE)**

**Beschreibung**

Gegenstand der Erfindung ist ein neues Verfahren zur Herstellung von homogenen flüssigen Mischungen aus festen Parfümrohstoffen und die Verwendung dieser Mischungen zur Herstellung von lösungsmittelfreien Fertigparfümformulierungen.

Aus anwendungstechnischen Gründen (z.B. Stabilität, Geruchsintensität bzw. Substantivität) werden in der modernen Parfümindustrie in zunehmendem Maße Feststoffe eingesetzt. Insbesondere für Waschmittel- und Weichspüler-Parfüms sind diese Stoffe unerläßlich, da diese Produkte bei der Herstellung, im Markt und später bei der Verarbeitung starken Beanspruchungen unterworfen sind. Der Duft dieser Produkte hat nicht nur eine ausschlaggebende Bedeutung bei Kaufentscheidungen für oder gegen ein bestimmtes Produkt, er begleitet vielmehr das Produkt von der Herstellung über die Lagerung bis zur Verwendung. Darüber hinaus muß die gewaschene bzw. weichgespülte Wäsche - auch nach Wochen der Aufbewahrung - einen angenehmen Geruch haben, um die hohen qualitativen Erwartungen des Käufers eines Markenartikels zu erfüllen.

Die Problematik dieser extremen Qualitätsanforderungen an ein modernes Fertigparfüm wird durch die Tatsache verdeutlicht, daß beim Wasch- bzw. Weichspülvorgang ca. 99,9 % des eingesetzten Industrieparfüms im Abwasser "verschwinden", während nur ca. 0,1 % auf die gewaschene Wäsche aufziehen.

Für die Mischung von Parfümrohstoffen werden üblicherweise die chemisch stabilen, flüssigen Anteile, in der Regel unter Zusatz weiterer Lösungsmittel wie Dipropylenglykol (DPG) und Phthalsäurediethylester (DEP) und ähnliche, vorgelegt und die festen Anteile nach und nach unter starkem Rühren zugegeben, wobei die Mischung von außen oder durch innenliegende Heizschlangen soweit erwärmt wird, daß die Feststoffe schmelzen bzw. in Lösung gehen. Da zur Erreichung eines ausreichenden Wärmetransports die Heizflächen auf höhere Temperaturen erhitzt werden müssen, tritt insbesondere bei empfindlichen Substanzen an diesen Heizflächen durch Überhitzen eine gewissen Schädigung ein. Darüber hinaus bewirkt die starke Durchmischung einen erhöhten Luftkontakt, so daß zur Vermeidung oxidativer Schädigungen unter einer kostenintensiven Schutzgasatmosphäre gearbeitet werden muß.

Nach der erfolgten Auflösung der Feststoffe wird die Mischung abgekühlt. Danach werden unter leichtem Rühren empfindliche, leicht flüchtige oder chemisch instabile Materialien zugesetzt und solange gerührt, bis die Mischung homogen ist. Diese Methode erfordert einen erheblichen Zeitaufwand, da am Anfang ein ungünstiges Mischverhältnis fest/flüssig vorliegt bzw. zwischendurch ein zeitraubender Heiz- und Kühlprozeß notwendig wird und die Verwendung zusätzlicher Lösungsmittel nicht nur Kosten verursacht und die Verarbeitungsmengen unnötig erhöht, sondern auch die Umwelt zusätzlich belastet.

In der DE-A 39 22 389.2 ist ein Verfahren beschrieben, bei dem das Eintragen der festen Rohstoffe in die flüssigen Stoffe unter gleichzeitiger Zugabe eines Schutzgases in die Rührzone erfolgt, wodurch die durch Überhitzung und mechanische Beanspruchung der empfindlichen Rohstoffe auftretende Schädigung absolut vermieden wird.

Als nachteilig erweist es sich jedoch, daß der Anteil der festen Stoffe an üblichen Parfümrezepturen vergleichsweise doch hoch ist, und daß solche Stoffe schlecht dosiert und gefördert werden können und zudem auch als Pulver große Volumina einnehmen, was Transport- und Lagerprobleme aufwirft. Als Ausweg werden solche Stoffe daher teilweise in geeigneten Lösungsmitteln aufgelöst und dadurch in flüssigen Zustand überführt, wodurch die Dosierung und das Handling vereinfacht wird. Dies hat jedoch den Nachteil, daß die Gesamtmenge sich durch die Lösungsmittelanteile stark vergrößert, wodurch Transport und Lagerhaltung erneut verteuert werden und zusätzlich Lösungsmittel in die Rezeptur eingebracht werden, welche nicht nur selbst einen Kostenfaktor darstellen, sondern auch als umweltbelastend einzustufen sind.

Die vorgenannten Feststoffanteile von 20-60 % der Gesamtparfümmischung setzen sich aus jeweils ca. 20 verschiedenen Rohstoffen zusammen, die ihrerseits aus etwa 100 gebräuchlichen Materialien je nach der gewünschten Duftnote ausgewählt werden. 5 dieser Stoffe, nämlich 6-Acetyl-1,1,3,4,4,6-hexamethylte-trahydro-naphthalin, $\alpha$,n-Hexylzimtaldehyd, p.t.-Butyl-methylhydrozimtaldehyd, p.-Methoxyacetophenon, Benzyl-o-hydroxybenzoat, die in fast allen Rezepturen allerdings in verschiedenen Anteilen vorkommen, machen dabei bereits über 70 % der Gesamtmenge aus. Zusammen mit den 10 nächsthäufigen Stoffen steigt der Anteil bereits auf über 90 %. Die mengenmäßig nächsten 10 Stoffe erhöhen den Anteil auf über 95 %. Alle übrigen Stoffe kommen lediglich in Mengen von unter 0,5 % vor, so daß ihre Zumischung unproblematisch ist.

Um die Nachteile bei der Verarbeitung von Feststoffen zu vermeiden, stellte sich daher die Aufgabe, diese Stoffe oder doch zumindest die Hauptkomponenten in flüssigen Zustand zu überführen und in dieser Form auch über längere Lagerung für den endgültigen Mischprozeß bereitzuhalten.

Eine längerfristige Lagerung oberhalb des jeweiligen Schmelzpunktes verbietet sich nicht nur wegen der Kosten, sondern vor allem aus Stabilitäts- und Qualitätsgründen. Die bei mengenmäßig unbedeutenden Komponenten teilweise praktizierte Lösung in "geruchsneutralen" Lösungsmitteln, wie Diethylphthalat (DEP) oder Dibutylglykol, Dipropylenglykol (DPG) und ähnliche Lösungsmittel, verbietet sich bei mengenmäßig bedeutenden Produkten natürlich, weil dadurch zu viel umwelt- und produktschädliche Lösungsmittel in die Rezeptur eingeführt werden.

Ein vorheriges Lösen in den "flüssigen" Bestandteilen der Rezepturen ist nicht möglich, da diese, soweit sie in größeren Mengen vorkommen, beispielsweise Phenylethylalkohol, schlechte Lösungsmittel sind oder nur in kleineren und für die verschiedenen Rezepturen wechselnden Mengenverhältnissen eingesetzt werden, so daß eine Vorablösung wegen der damit verbundenen Lagerungs- und Logistikprobleme nicht in Frage kommt.

Aus der US-A 4,650,603 sind bereits flüssige Mischungen aus obigem, tricyclischen Isochromanderivaten mit festen Tetrahydronaphthalinderivaten bekannt, welche als Aromaverstärker in Parfümmischungen und Waschmitteln eingesetzt werden sollen, und über einen weiten Konzentrationsbereich einen Schmelzpunkt von unter 20 ° C aufweisen.

Überraschender Weise wurde nun gefunden, daß sich die vorgenannten festen oder viskosen Hauptprodukte zu binären und ternären Mischungen (Duftbausteinen) kombinieren lassen, welche nicht nur bei Raumtemperatur, sondern teilweise auch bis 0 ° C oder -20 ° C dünnflüssig sind, und daß durch Zusammenfügen dieser Duftbausteine in geeigneten Mengenverhältnissen praktisch alle gebräuchlichen Industrieparfümrezepturen ohne Zugabe von Lösungsmitteln hergestellt werden können.

Die erfindungsgemäßen flüssigen Parfümrohstoffmischungen (Duftbaustoffe) bestehen demgemäß aus einer Mischung, welche aus 2 oder 3 der folgenden Stoffe gebildet ist:

a) dem festen 6-Acetyl-1,1,3,4,4,6-hexamethyl-tetrahydro-naphthalin,
b) dem festen oder öligen $\alpha$-n-Hexylzimtaldehyd,
c) dem öligen p.t.-Butyl-$\alpha$-methylhydrozimtaldehyd,
d) dem festen p.-Methoxyacetophenon und
e) dem festen Benzyl-$\alpha$-hydroxybenzoat,

wobei bei binären Mischungen 10 - 90 % und bei ternären 10 - 70 % der einzelnen Stoffe enthalten sind.

Zur Herstellung der homogenen Parfüme wurden aus den Komponenten a)-e) zunächst die bei Raumtemperaturen flüssigen binären oder ternären Vormischungen gebildet und in diese Mischungen die übrigen festen und flüssigen Produkte gleichzeitig oder in einer für die jeweilige Mischung beliebigen, von der chemischen und physikalischen Natur der Stoffe unabhängigen Reihenfolge eingetragen. Bei der Verarbeitung wird die Mischung auf einer Temperatur zwischen dem Schmelzpunkt der eutektischen Vormischung und der Schmelztemperatur der Feststoffe gehalten. Die erforderliche Schmelz- bzw. Lösungswärme der festen Stoffe wird mit einem Ultraschall- oder Mikrowellengenerator zugeführt.

Zur Herstellung des fertigen Parfüms werden vorzugsweise mehrere Vormischungen verschiedener Zusammensetzung als Duftbausteine zu der gewünschten Rezeptur zusammengefügt oder ggf. durch Zufügen weiterer Parfümstoffe modifiziert. Die Zugabe der flüssigen eutektischen Mischungen und anderen Parfümrohstoffe in einem automatischen Mischprozeß computergesteuert ohne Zusatz von Lösungsmitteln durchgeführt wird.

Diese flüssigen Mischungen, die bei Temperaturen von 30-80°C unter dem Schmelzpunkt der Komponenten, d.h. teilweise bei -20° bis 0°C, noch flüssig sind, werden im folgenden als "eutektische Mischungen" bezeichnet, um auf den gegenüber den Einzelkomponenten unerwartet niedrigen Festpunkt hinzuweisen. Ob die jeweiligen festen Phasen sich mischen oder nicht, d.h. ob echte Eutektika vorliegen, wurde dabei nicht untersucht und berücksichtigt.

Eigenschaften der Rohstoffe

## 1. 6-Acetyl-1,1,3,4,4,6-hexamethyltetrahydronaphthalin (AHN)

Weißes kristallines Pulver, Schmelzpunkt (nach Reinheitsgrad) 55-60°C, handelsübliche Reinheit 95-99,8 %, sehr empfindlich Fremdgerüche zu binden, hohe Substantivität, relativ schwer löslich in flüssigen Parfümrohstoffen, leicht löslich in Diethylphthalat und Alkohol, **Moschusgeruch**.

## 2. $\alpha$-Hexylcinnamic Aldehyde (HCA)

Feststoff bei 10-23°C. Bei höherer Temperatur gelbliche, ölige Flüssigkeit, die stark zur Oxidation (Zimtsäure) neigt. Löst sich gut in Diethylphthalaten und Dipropylenglykol, **blumige, fruchtige Geruchsnote**.

### 3. Benzylsalicylat (BSA)

Kristalline Masse, die bei ca. 25-30°C zur farblosen öligen Substanz schmilzt. Löslich in Diethylphthalat, schlecht löslich in Dipropylenglykol. **Süßblumiger, leicht balsamischer Geruch** von geringer Intensität.

### 4. p.Methoxyacetophenon oder Methyl-4-methoxyphenylketon (PMA)

Farblose, zur Klumpenbildung neigende Kristalle. Schmelzpunkt 36-40°C. Typisch **blumig-animalisch stark intensiver Geruch**, schwer löslich in flüssigen Parfümrohstoffen, Dipropylenglykol, leicht löslich in Diethylphthalate und Alkohol.

### 5. p.t.-Butyl-$\alpha$-methyl-hydrocinnamic-aldehyd (PTB)

Farblose, ölige Flüssigkeit, löst sich in Alkohol und anderen Parfümölen und Diethylphthalate. **Frischer aldehydischer Blumengeruch**, sehr oxidationsempfindlich, erstarrt dabei zur festen Carbonsäure (Zimtsäurederivat), Risiko zu Autoxidation und Selbstentzündung, wird im Gemisch mit anderen geeigneten Parfümrohstoffen eliminiert.

Die vorstehend in Klammern beigefügten Kurzbezeichnungen werden im weiteren Text verwendet.

Als Zwei-(binäre) oder Mehrstoffgemische zeigen die erfindungsgemäß vorgeschlagenen Gemische der erwähnten 5 Einzelmaterialien in weiten Mischungsverhältnissen eine überraschend starke Schmelzpunktdepression verbunden mit einer ausgezeichneten Temperaturstabilität bzw. chemischen Stabilität gegen Oxidation und Polymerisation. In allen Fällen entstehen durch das Mischen von zwei oder mehr der genannten Substanzen in den vorgegebenen Mischverhältnissen stabile Flüssigkeiten. Diese können als "Duftbausteine" mit den übrigen flüssigen Rohstoffen ohne weitere Vorbehandlung bei Temperaturen von 10-20° unproblematisch über Pumpen und Ventile dosiert und in einfachen Mischern zu Fertigparfümrezepturen weiterverarbeitet werden.

Teilweise ergab sich eine überraschende "Superstabilität", da einige Mischungen selbst bei längerer Lagerung in Temperaturbereichen von minus 18° bis minus 20°C noch flüssig (leichtflüssig bis mittelviskos) bleiben. Auch bei Zugabe von Impfkristallen kristallisieren diese Mischungen nicht aus. Die erzielten Einzelergebnisse sind den nachfolgenden Tabellen zu entnehmen, wobei folgende Klassifikation verwendet wurde:

1 = erstarrt direkt nach Abkühlen
2 = erstarrt ganz nach einem Tag
3 = erstarrt teilweise nach einem Tag
4 = erstarrt ganz nach 3 Tagen
5 = erstarrt teilweise nach 3 Tagen
6 = erstarrt nicht nach 3 Tagen

Teilweise wurde die Viskosität bei 20°C gemessen. Entsprechende Werte in mPa•s sind ebenfalls in die folgenden Tabellen eingefügt.

| Mischung aus 6-Acetyl-1,1,3,4,4,6-hexamethyltetrahydronaphthalin (AHN), Hexylzimtaldehyd (HCA), pt-Butyl-$\alpha$-methylhydrozimtaldehyd (PTB) | | | | | | | |
|---|---|---|---|---|---|---|---|
| AHN | Teile HCA | PTB | Visk. | -20 °C | 0 °C | + 10 °C | + 20 °C |
| 40 | 10 | 50 | | 5 | 5 | 6 | 6 |
| 40 | 20 | 40 | | 6 | 6 | 6 | 6 |
| 40 | 30 | 30 | 32 | 6 | 6 | 6 | 6 |
| 40 | 40 | 20 | | 6 | 6 | 6 | 6 |
| 40 | 50 | 10 | | 4 | 4 | 6 | 6 |
| 50 | 5 | 45 | | 4 | 4 | 6 | 6 |
| 50 | 15 | 35 | 42 | 6 | 6 | 6 | 6 |
| 50 | 25 | 25 | 42 | 6 | 6 | 6 | 6 |
| 50 | 35 | 15 | | 4 | 4 | 5 | 6 |
| 50 | 45 | 5 | | 4 | 4 | 5 | 6 |
| 60 | 10 | 30 | | 6 | 6 | 6 | 6 |
| 60 | 20 | 20 | 53 | 6 | 6 | 6 | 6 |
| 60 | 30 | 10 | | 4 | 5 | 5 | 6 |

| Mischung aus 6-Acetyl-1,1,3,4,4,6-hexamethyltetrahydronaphthalin (AHN) und p.Methoxyacetophenon (PMA) | | | | | | |
|---|---|---|---|---|---|---|
| AHN | Teile PMA | Visk. | -20 °C | 0 °C | + 10 °C | + 20 °C |
| 25 | 75 | | 1 | 2 | 2 | 5 |
| 50 | 50 | 27 | 1 | 2 | 3 | 6 |
| 75 | 25 | | 1 | 2 | 2 | 5 |

| Mischung aus 6-Acetyl-1,1,3,4,4,6-hexamethyltetrahydronaphthalin (AHN) und Hexylzimtaldehyd (HCA) | | | | | | |
|---|---|---|---|---|---|---|
| AHM | Teile HCA | Visk. | -20 °C | 0 °C | + 10 °C | + 20 °C |
| 90 | 10 | | 1 | 1 | 1 | 1 |
| 80 | 20 | | 1 | 1 | 4 | 4 |
| 70 | 30 | | 1 | 4 | 6 | 6 |
| 60 | 40 | 30 | 1 | 5 | 6 | 6 |
| 50 | 50 | 40 | 1 | 5 | 6 | 6 |
| 40 | 60 | | 2 | 6 | 6 | 6 |
| 30 | 70 | | 5 | 5 | 6 | 6 |
| 20 | 80 | 100 | 1 | 5 | 5 | 6 |
| 10 | 90 | | 1 | 4 | 5 | 6 |

| Mischung aus 6-Acetyl-1,1,3,4,4,6-hexamethyltetrahydronaphthalin (AHN) und Benzylsalicylat (BSA) | | | | | | |
|---|---|---|---|---|---|---|
| AHN | Teile BSA | Visk. | -20 °C | 0 °C | +10 °C | +20 °C |
| 90 | 10 | | 1 | 1 | 1 | 1 |
| 80 | 20 | | 1 | 1 | 1 | 2 |
| 70 | 30 | | 2 | 2 | 4 | 5 |
| 60 | 40 | | 4 | 4 | 5 | 5 |
| 50 | 50 | | 4 | 4 | 5 | 6 |
| 40 | 60 | 40 | 5 | 5 | 6 | 6 |
| 30 | 70 | 32 | 5 | 6 | 6 | 6 |
| 20 | 80 | | 5 | 5 | 6 | 6 |
| 10 | 90 | | 2 | 4 | 4 | 6 |

| Mischung aus Hexylzimtaldehyd (HCA) und Benzylsalicylat (BSA) | | | | | | |
|---|---|---|---|---|---|---|
| HCA | Teile BSA | Visk. | -20 °C | 0 °C | +10 °C | +20 °C |
| 90 | 10 | | 2 | 4 | 5 | 6 |
| 80 | 20 | | 2 | 4 | 5 | 6 |
| 70 | 30 | | 4 | 5 | 6 | 6 |
| 60 | 40 | | 4 | 5 | 6 | 6 |
| 50 | 50 | 20 | 5 | 6 | 6 | 6 |
| 40 | 60 | 20 | 4 | 6 | 6 | 6 |
| 30 | 70 | | 2 | 5 | 5 | 6 |
| 20 | 80 | | 2 | 5 | 5 | 6 |
| 10 | 90 | | 2 | 4 | 5 | 6 |

| Mischung aus Benzylsalicylat (BSA) und para-Methoxyacetophenon (PMA) | | | | | | |
|---|---|---|---|---|---|---|
| BSA | Teile PMA | Visk. | -20 °C | 0 °C | +10 °C | +20 °C |
| 20 | 80 | | 2 | 2 | 4 | 4 |
| 50 | 50 | 17 | 2 | 5 | 6 | 6 |
| 66,6 | 33,3 | | 4 | 6 | 6 | 6 |
| 80 | 20 | | 2 | 6 | 6 | 6 |

| Mischung aus Hexylzimtaldehyd (HCA) und para-Methoxyaceto-phenon (PMA) | | | | | | |
|---|---|---|---|---|---|---|
| HCA | Teile PMA | Visk. | -20 °C | 0 °C | +10 °C | +20 °C |
| 80 | 20 | | 4 | 6 | 6 | 6 |
| 60 | 40 | 15 | 4 | 5 | 6 | 6 |
| 40 | 60 | | 4 | 3 | 5 | 5 |
| 20 | 80 | | 1 | 2 | 2 | 4 |

| Mischung aus 6-Acetyl-1,1,3,4,4,6-hexamethyltetrahydronaphthalin (AHN), Hexylzimtaldehyd (HCA) und Benzylsalicylat (BSA) | | | | | | | |
|---|---|---|---|---|---|---|---|
| AHN | Teile HCA | BSA | Visk. | -20 °C | 0 °C | +10 °C | +20 °C |
| 40 | 10 | 50 | | 4 | 4 | 5 | 6 |
| 40 | 20 | 40 | | 4 | 5 | 6 | 6 |
| 40 | 30 | 30 | 30 | 6 | 6 | 6 | 6 |
| 40 | 40 | 20 | | 6 | 6 | 6 | 6 |
| 40 | 50 | 10 | 30 | 6 | 6 | 6 | 6 |
| 50 | 5 | 45 | | 4 | 4 | 5 | 6 |
| 50 | 15 | 35 | 45 | 4 | 4 | 5 | 6 |
| 50 | 25 | 25 | | 4 | 5 | 6 | 6 |
| 50 | 35 | 15 | 40 | 6 | 6 | 6 | 6 |
| 50 | 45 | 5 | | 6 | 6 | 6 | 6 |
| 60 | 10 | 30 | | 2 | 4 | 5 | 6 |
| 60 | 20 | 20 | | 2 | 4 | 5 | 6 |
| 60 | 30 | 10 | | 2 | 4 | 5 | 6 |

Herstellung der eutektischen Mischungen

Die Herstellung der flüssig-stabilen Materialmischungen ist völlig unproblematisch. Als besonders vorteilhaft erweist sich hier, wenn eine oder mehrere der Komponenten in flüssiger Form vorliegen und diese im vorgesehenen Verhältnis direkt zusammengegeben werden. Da alle Einzelkomponenten direkt nach deren Synthese in flüssiger Form vorliegen, ist die Herstellung der eutektischen Mischungen direkt bei Rohstoffherstellern besonders energiesparend und einfach.

Es ist jedoch auch möglich, die Mischungen erst beim Benutzer herzustellen, indem vorgeschmolzene stabile Einzelkomponenten vorgelegt und im vorgesehenen Verhältnis mit kristallinen Materialien gemischt werden.

Die Zugabe der flüssigen Duftbausteine zur Restformel erfolgt ohne weitere Vorbehandlung bei Raumtemperatur durch einfaches Zupumpen oder über Prozeßcomputer gesteuert über Dosierventile. Hiermit können Bereiche des Parfümmischprozesses automatisiert werden, deren Automatisierung bisher aus technischen Gründen nicht möglich war.

Die homogene Endmischung wird in allen Fällen nach erfolgter Zugabe durch einen kurzen Rührprozeß oder durch Zirkulation/Umpumpen erfolgen.

Die im klassischen Parfümprozeß zum Vorlösen der hohen Feststoffanteile eingesetzten geruchlosen klassischen Lösungsmittel (Diethylphthalat, Dipropylenglykol etc.) erwiesen sich als überflüssig, wenn vorverflüssigte Feststoffgemische zum Einsatz kommen. Da in diesem Fall bei Raumtemperatur gearbeitet wird, können die oxidations- bzw. polymerisationsempfindlichen oder leicht flüchtigen Anteile der Parfümformel in frei wählbarer Zugabenreihenfolge beliebig zugegeben werden.

Da nach dem erfindungsgemäß vorgeschlagenen Verfahren nur noch Flüssigkeiten eingesetzt werden, deren Homogenisierung völlig unproblematisch ist, werden erhebliche Kapazitätssteigerungen gegenüber klassischen Parfümmischprozessen ermöglicht.

Die erfindungsgemäß vorgeschlagenen flüssigen eutektischen Mischungen aus den erwähnten Einzelkomponenten lassen sich als Duftbausteine in folgenden Variationen einsetzen.

| BSA | HCA | PTB | AHC | PMA |
|---|---|---|---|---|
| süß flo- ral, bal- samisch | süß floral leicht fruchtig | süß floral aldehydisch grün | Moschus | süß floral holzig animalisch |

frisch, floral, fruchtig aldehydisch grün

frisch, floral, alde- hydisch grüne Moschus- note

süß, floral, balsamische Moschusnote

frisch, florale, süße Moschusnote mit holzig- animalischem Hintergrund

reich, florale, aldehydisch frische Moschusnote mit balsa- misch/holzig-animalischem Hinter- grund.

Obige Duftbausteine können als Grundbausteine einer modernen Industrieparfümformel angesehen werden, die in den unterschiedlichsten Variationen eingesetzt ca. 20-50 % der Gesamtformel oder 70-90 % der Feststoffe ausmachen. Alle bisher getesteten Formelvariationen waren bei Raumtemperatur flüssig.

Die Herstellung von weiteren erfolgreichen Duftbausteinen auf der Basis des vorgeschlagenen Grundbausteines ist durch Zufügen weiterer Bestandteile wie folgt denkbar und in allen Variationen denkbar.

Ionone, γ-Methylionone
und andere Derivate
warm, holzig, balsamisch,
floral

Benzylacetat
Benzylaceton
frisch, floral
grün, fruchtig

Eugenol, florale
warme gewürzige
Moschusnote mit
floralem Hintergrund

GRUNDBAUSTEIN

Paratertiary
Butylcyclohexylacetate, florale
holzig cremige
aldehydisch
frische Moschusnote

Orangenöle
Citronenöle
Geraniol
Citronellol
und andere Derivate
Tricyclodecenylacetate
Tricyclodecenylpropionate
Phenylethylalkohol
aldehyd. frische, rosig
reiche, blumige Moschusnote mit holzigem/grünen
animalischen Hintergrund

Linalool, Lavendelöle,
Linalylacetat und Derivate, floral fruchtiger
Moschusnote mit feinem
frischen Zitrushintergrund

Der Anteil dieser aus wenigen Grundkomponenten zusammengesetzten Duftbausteine an allen aktuellen Industrieparfümformeln beträgt zwischen 40 und 90 %. Die übrigen entweder flüssigen oder in geringen Mengen einzubringenden,festen Stoffe lassen sich damit problemlos mischen.

Die nach den erfindungsgemäß vorgeschlagenen Verfahren hergestellten Fertigparfümmischungen erwiesen sich überraschenderweise auch ohne Lösungsmittelanteile als "superstabil bei Festtemperaturen von 0° bis minus 20°C". Damit kann in Zukunft die kostspielige Lagerung von Fertigparfüms in beheizten Lagerräumen ohne jedes Qualitätsrisiko vermieden werden.

**Patentansprüche**

1. Verfahren zur Herstellung von homogenen Mischungen aus flüssigen und/oder festen Parfümrohstoffen durch Mischen der Bestandteile und Verflüssigen der Mischung, **dadurch gekennzeichnet,** daß aus

   a) dem festen 6-Acetyl-1,1,3,4,4,6-hexamethyl-tetrahydro-naphthalin,

   b) dem festen oder öligen α,n-Hexylzimtaldehyd,

   c) dem öligen p.t.-Butyl-α-methylhydrozimtaldehyd,

   d) dem festen p.-Methoxyacetophenon und

   e) dem festen Benzyl-α-hydroxybenzoat,

   eine bei Raumtemperatur flüssige binäre oder ternäre eutektische Vormischung gebildet wird, welche bei binären Mischungen 10-90 % und bei ternären 10-70 % der einzelnen Stoffe enthält und in diese Mischung die übrigen festen und flüssigen Produkte gleichzeitig oder in einer für die jeweilige Mischung beliebigen, von der chemischen und physikalischen Natur der Stoffe unabhängigen Reihenfolge eingetragen werden.

2. Verfahren gemäß Anspruch 1, **dadurch gekennzeichnet,** daß die Mischung auf einer Temperatur zwischen dem Schmelzpunkt der eutektischen Vormischung und der Schmelztemperatur der Feststoffe gehalten wird.

3. Verfahren nach einem der Ansprüche 1 bis 2, **dadurch gekennzeichnet,** daß die Schmelz- bzw. Lösungswärme der festen Stoffe mit einem Ultraschall- oder Mikrowellengenerator zugeführt wird.

4. Verfahren nach einem der Ansprüche 1 bis 3, **dadurch gekennzeichnet,** daß mehrere Vormischungen verschiedener Zusammensetzung als Duftbausteine zu der gewünschten Rezeptur zusammengefügt werden.

5. Verfahren nach einem der Ansprüche 1 bis 4, **dadurch gekennzeichnet,** daß die Duftbausteine durch Zufügen weiterer Parfümstoffe modifiziert sind.

6. Verfahren nach einem der Ansprüche 1 bis 5, **dadurch gekennzeichnet,** daß die Zugabe der flüssigen eutektischen Mischungen und anderen Parfümrohstoffe in einem automatischen Mischprozeß computergesteuert ohne Zusatz von Lösungsmitteln durchgeführt wird.

7. Eutektische Parfümvormischung **dadurch gekennzeichnet,** daß sie aus einer bei Raumtemperatur flüssigen binären oder ternären eutektischen Mischung besteht, welche aus 2 oder 3 der folgenden Stoffen gebildet ist:
   a) dem festen 6-Acetyl-1,1,3,4,4,6-hexamethyl-tetrahydro-naphthalin,
   b) dem festen oder öligen $\alpha$,n-Hexylzimtaldehyd,
   c) dem öligen p.t.-Butyl-$\alpha$-methylhydrozimtaldehyd,
   d) dem festen p.-Methoxyacetophenon und
   e) dem festen Benzyl-$\alpha$-hydroxybenzoat,
   wobei bei binären Mischungen 10-90 % und bei ternären 10-70 % der einzelnen Stoffe enthalten sind.

8. Eutektische Parfümvormischungen gemäß Anspruch 7, **dadurch gekennzeichnet,** daß sie 6-Acetyl-1,1,3,4,4,6-hexamethyltetrahydro-naphthalin und $\alpha$,n-Hexylzimtaldehyd enthalten.

9. Eutektische Parfümvormischungen gemäß **Anspruch 7, dadurch gekennzeichnet,** daß sie 6-Acetyl-1,1,3,4,4,6-hexamethyltetrahydro-naphthalin und p.t.-Butyl-$\alpha$-methylhydrozimtaldehyd enthalten.

10. Eutektische Parfümvormischungen gemäß Anspruch 7**, dadurch gekennzeichnet, daß** sie 6-Acetyl-1,1,3,4,4,6-hexamethyltetrahydro-naphthalin und p.-Methoxyacetophenon enthalten.

11. Eutektische Parfümvormischungen gemaß Anspruch 7, **dadurch gekennzeichnet,** daß sie 6-Acetyl-1,1,3,4,4,6-hexamethyltetrahydro-naphthalin und Benzyl-o-hydroxybenzoat enthalten.

12. Eutektische Parfümvormischungen gemaß Anspruch 7, **dadurch gekennzeichnet,** daß sie $\alpha$,n-Hexylzimtaldehyd und p.t.-Butyl-$\alpha$-methylhydrozimtaldehyd enthalten.

13. Eutektische Parfümvormlschungen gemäß Anspruch 7, **dadurch gekennzeichnet,** daß sie $\alpha$,n-Hexylzimtaldehyd und p.-Methoxyacetophenon enthalten.

14. Eutektische Parfümvormischungen gemaß Anspruch 7, **dadurch gekennzeichnet,** daß sie $\alpha$,n-Hexylzimtaldehyd und Benzyl-o-hydroxybenzoat enthalten.

15. Eutektische Parfümvormischungen gemäß Anspruch 7, **dadurch gekennzeichnet,** daß sie p.t.-Butyl-$\alpha$-methylhydrozimtaldehyd und p.-Methoxyacetophenon enthalten.

16. Eutektische Parfümvormischungen gemäß Anspruch 7, **dadurch gekennzeichnet,** daß sie p.t.Butyl-$\alpha$-methylhydrozimtaldehyd und Benzyl-o-hydroxybenzoat enthalten.

17. Eutektische Parfümvormischungen gemäß Anspruch 7, **dadurch gekennzeichnet,** daß sie p.-Methoxyacetophenon und Benzyl-o-hydroxybenzoat enthalten.

18. Eutektische Parfümvormischungen gemäß Anspruch 7, **dadurch gekennzeichnet,** daß sie 6-Acetyl-1,1,3,4,4,6-hexamethyltetrahydronaphthalin, $\alpha$,n-Hexylzimtaldehyd und Benzylsalicylat enthalten.

## Claims

1. Process for the production of homogeneous mixtures of liquid and/or solid perfume raw materials by mixing of the components and liquefaction of the mixture, characterised in that from

10

a) solid 6-acetyl-1,1,3,4,4,6-hexamethyltetrahydronaphthalene,

b) solid or oily $\alpha$,n-hexylcinnamaldehyde,

c) oily p-t.-butyl-$\alpha$-methylhydrocinnamaldehyde,

d) solid p-methoxyacetophenone and

e) solid benzyl $\alpha$-hydroxybenzoate

there is formed a binary or ternary eutectic mixture liquid at room temperature which, in the case of binary mixtures, contains 10 - 90% and, in the case of ternary mixtures, 10 - 70% of the individual materials and into this mixture are introduced the remaining solid and liquid products simultaneously or in a sequence as desired for the mixture in question, independently of the chemical and physical nature of the materials.

2. Process according to claim 1, characterised in that the mixture is kept at a temperature between the melting point of the eutectic premixture and the melting temperature of the solid materials.

3. Process according to one of claims 1 to 2, characterised in that the melting or dissolving heat of the solid materials is introduced with an ultrasonic or microwave generator.

4. Process according to one of claims 1 to 3, characterised in that several premixtures of different composition are mixed together to the desired formulation as scent components.

5. Process according to one of claims 1 to 4, characterised in that scent components are modified by addition of further perfume materials.

6. Process according to one of claims 1 to 5, characterised in that the addition of the liquid eutectic mixtures and of other perfume raw materials is carried out in an automatic computer-controlled mixing process without addition of solvents.

7. Eutectic perfume premixture, characterised in that it consists of a binary or ternary eutectic mixture liquid at room temperature which is formed from 2 or 3 of the following materials:

a) solid 6-acetyl-1,1,3,4,4,6-hexamethyltetrahydronaphthalene,

b) solid or oily $\alpha$,n-hexylcinnamaldehyde,

c) oily p-t.-butyl-$\alpha$-methylhydrocinnamaldehyde,

d) solid p-methoxyacetophenone and

e) solid benzyl $\alpha$-hydroxybenzoate,

whereby, in the binary mixtures, are contained 10 - 90% and, in the ternary mixtures, 10 - 70% of the individual materials.

8. Eutectic perfume premixtures according to claim 7, characterised in that they contain 6-acetyl-1,1,3,4,4,6-hexamethyltetrahydronaphthalene and $\alpha$,n-hexylcinnamaldehyde.

9. Eutectic perfume premixtures according to claim 7, characterised in that they contain 6-acetyl-1,1,3,4,4,6-hexamethyltetrahydronaphthalene and p-y.-butyl-$\alpha$-methylhydrocinnamaldehyde.

10. Eutectic perfume premixtures according to claim 7, characterised in that they contain 6-acetyl-1,1,3,4,4,6-hexamethyltetrahydronaphthalene and p-methoxyacetophenone.

11. Eutectic perfume premixtures according to claim 7, characterised in that they contain 6-acetyl-1,1,3,4,4,6-hexamethyltetrahydronaphthalene and benzyl o-hydroxybenzoate.

12. Eutectic perfume premixtures according to claim 7, characterised in that they contain $\alpha$-n-hexylcinnamaldehyde and p-t.-butyl-$\alpha$-methylhydrocinnamaldehyde.

13. Eutectic perfume premixtures according to claim 7, characterised in that they contain $\alpha$,n-hexylcinnamaldehyde and p-methoxyacetophenone.

14. Eutectic perfume premixtures according to claim 7, characterised in that they contain $\alpha$,n-hexylcinnamaldehyde and benzyl o-hydroxybenzoate.

**15.** Eutectic perfume premixtures according to claim 7, characterized in that they contain p-t.-butyl-α-methylhydrocinnamaldehyde and p-methoxyacetophenone.

**16.** Eutectic perfume premixtures according to claim 7, characterized in that they contain p-t.-butyl-α-methylhydrocinnamaldehyde and benzyl o-hydroxybenzoate.

**17.** Eutectic perfume premixtures according to claim 7, characterized in that they contain p-methoxyacetophenone and benzyl o-hydroxybenzoate.

**18.** Eutectic perfume premixtures according to claim 7, characterised in that they contain 6-acetyl-1,1,3,4,4,6-hexamethyltetrahydronaphthalene, α,n-hexylcinnamaldehyde and benzyl salicylate.

**Revendications**

**1.** Procédé de préparation de mélanges homogènes constitués de matières premières de parfums liquides et/ou solides par mélange des constituants et par liquéfaction du mélange, caractérisé en ce qu'un prémélange eutectique, binaire ou ternaire, liquide à température ambiante est formé à partir de
a) 6-actéyl-1,1,3,4,4,6-hexaméthyltétrahydronaphtalène solide,
b) aldéhyde α,n-hexylcinnamique huileux ou solide,
c) aldéhyde p-t.-butyl-α-méthylhydrocinnamique huileux,
d) p-méthoxyacétophénone solide et
e) α-hydroxybenzoate de benzyle solide,
qui contient dans le cas des mélanges binaires 10 - 90 % et dans le cas des mélanges ternaires 10 - 70 % des matières individuelles et qu'on introduit dans ce mélange les autres produits solides et liquides simultanément ou dans un ordre indépendant de la nature chimique et physique des matières et quelconque pour le mélange respectif.

**2.** Procédé selon la revendication 1, caractérisé en ce qu'on maintient le mélange à une température entre le point de fusion du prémélange eutectique et la température de fusion des matières solides.

**3.** Procédé selon l'une des revendications 1 à 2, caractérisé en ce qu'on fournit la chaleur de fusion, respectivement de mise en solution, des matières solides grâce à un générateur d'ultrasons ou de micro-ondes.

**4.** Procédé selon l'une quelconque des revendications 1 à 3, caractérisé en ce qu'on combine plusieurs prémélanges de compositions différentes en tant qu'unités constitutives parfumées en formulation souhaitée.

**5.** Procédé selon l'une quelconque des revendications 1 à 4, caractérisé en ce que les unités constitutives parfumées sont modifiées par addition de matières parfumées supplémentaires.

**6.** Procédé selon l'une quelconque des revendications 1 à 5, caractérisé en ce que l'addition des mélanges eutectiques liquides et des autres matières premières de parfums est réalisée dans un procédé de mélange automatique commandé par ordinateur, sans addition de solvants.

**7.** Prémélange eutectique de parfums, caractérisé en ce qu'il est constitué d'un mélange eutectique binaire ou ternaire, liquide à température ambiante, qui est formé par deux ou trois des matières suivantes :
a) 6-actéyl-1,1,3,4,4,6-hexaméthyltétrahydronaphtalène solide,
b) aldéhyde α,n-hexylcinnamique huileux ou solide,
c) aldéhyde p-t.-butyl-α-méthylhydrocinnamique huileux,
d) p-méthoxyacétophénone solide et
e) α-hydroxybenzoate de benzyle solide,
dans lequel sont contenus dans le cas de mélanges binaires 10 à 90 % et dans le cas de mélanges ternaires 10 à 70 % des matières individuelles.

**8.** Prémélanges eutectiques de parfums selon la revendication 7, caractérisés en ce qu'ils contiennent du 6-acétyl-1,1,3,4,4,6-hexaméthyltétrahydronaphtalène et de l'aldéhyde α,n-hexylcinnamique.

9. Prémélanges eutectiques de parfums selon la revendication 7, caractérisés en ce qu'ils contiennent du 6-acétyl-1,1,3,4,4,6-hexaméthyltétrahydronaphtalène et de l'aldéhyde p-t.-butyl-$\alpha$-méthylhydrocinnamique.

10. Prémélanges eutectiques de parfums selon la revendication 7, caractérisés en ce qu'ils contiennent du 6-acétyl-1,1,3,4,4,6-hexaméthyltétrahydronaphtalène et de la p-méthoxyacétophénone.

11. Prémélanges eutectiques de parfums selon la revendication 7, caractérisés en ce qu'ils contiennent du 6-acétyl-1,1,3,4,4,6-hexaméthyltétrahydronaphtalène et de l'o-hydroxybenzoate de benzyle.

12. Prémélanges eutectiques de parfums selon la revendication 7, caractérisés en ce qu'ils contiennent de l'aldéhyde $\alpha$,n-hexylcinnamique et de l'aldéhyde p-t.-butyl-$\alpha$-méthylhydrocinnamique.

13. Prémélanges eutectiques de parfums selon la revendication 7, caractérisés en ce qu'ils contiennent de l'aldéhyde $\alpha$,n-hexylcinnamique et de la p-méthoxyacétophénone.

14. Prémélanges eutectiques de parfums selon la revendication 7, caractérisés en ce qu'ils contiennent de l'aldéhyde $\alpha$,n-hexylcinnamique et de l'o-hydroxybenzoate de benzyle.

15. Prémélanges eutectiques de parfums selon la revendication 7, caractérisés en ce qu'ils contiennent de l'aldéhyde p-t.-butyl-$\alpha$-méthylhydrocinnamique et de la p-méthoxyacétophénone.

16. Prémélanges eutectiques de parfums selon la revendication 7, caractérisés en ce qu'ils contiennent de l'aldéhyde p-t.-butyl-$\alpha$-méthylhydrocinnamique et de l'o-hydroxybenzoate de benzyle.

17. Prémélanges eutectiques de parfums selon la revendication 7, caractérisés en ce qu'ils contiennent de la p-méthoxyacétophénone et de l'o-hydroxybenzoate de benzyle.

18. Prémélanges eutectiques de parfums selon la revendication 7, caractérisés en ce qu'ils contiennent du 6-acétyl-1,1,3,4,4,6,-hexaméthyltétrahydronaphtalène, de l'aldéhyde $\alpha$,n-hexylcinnamique et du salicylate de benzyle.